# EUROPEAN PATENT SPECIFICATION

(11) **EP 2 811 933 B1**
(45) Date of publication and mention of the grant of the patent: **26.07.2023**
(21) Application number: 13716384.6
(22) Date of filing: 08.02.2013
(51) Int. Cl.: A61B 34/20, A61B 90/00

(54) **SHAFT TRACKER FOR REAL-TIME NAVIGATION TRACKING**
SCHAFTVERFOLGER FÜR EINE ECHTZEITNAVIGATIONSVERFOLGUNG
SUIVEUR SUR TIGE POUR LE SUIVI DE LA NAVIGATION EN TEMPS RÉEL

(30) Priority: 09.02.2012 US 201261596749 P
(43) Date of publication of application: 17.12.2014
(73) Proprietor: Koninklijke Philips N.V., 5656 AG Eindhoven (NL)
(72) Inventor: LIU, Xin, NL-5656 AE Eindhoven (NL); CHIU, William, Wing, Nin, NL-5656 AE Eindhoven (NL); KRUECKER, Jochen, NL-5656 AE Eindhoven (NL); DALAL, Sandeep, M., NL-5656 AE Eindhoven (NL)
(74) Representative: Philips Intellectual Property & Standards
(86) International application number: PCT/IB2013/051050
(87) International publication number: WO 2013/118090

(56) References cited:
- DE-A1-102010 046 948
- US-A1- 2002 143 317
- US-A1- 2007 016 009
- US-A1- 2010 210 939

## Description

The present invention generally relates to an intervention instrument for minimally invasive interventions and surgeries. The present invention specifically relates to a shaft tracker integrated on a shaft of the intervention instrument for facilitating the tracking of the navigation of the intervention instrument.

Electromagnetic ("EM") tracking and optical tracking have proven to be useful tools for many minimally invasive interventions and surgeries. Specifically, intra-operative real-time imaging modalities (e.g., x-ray, endoscope and ultrasound) are linked with pre-operative imaging modalities (e.g., computed tomography and magnetic resonance imaging) via the aid of EM tracking or optical tracking whereby a pre-operative roadmap may be utilized to assist guidance to the real-time imaging. In addition, the instrument tip is dynamically tracked by having a tracking EM sensor coil or a tracking optical marker attached or embedded into the instrument itself whereby a physician may precisely localize the position and orientation of the instrument and its relationship to a target anatomical location based on the image fusion.

Conventionally, there are two approaches for enabling instrument tracking.

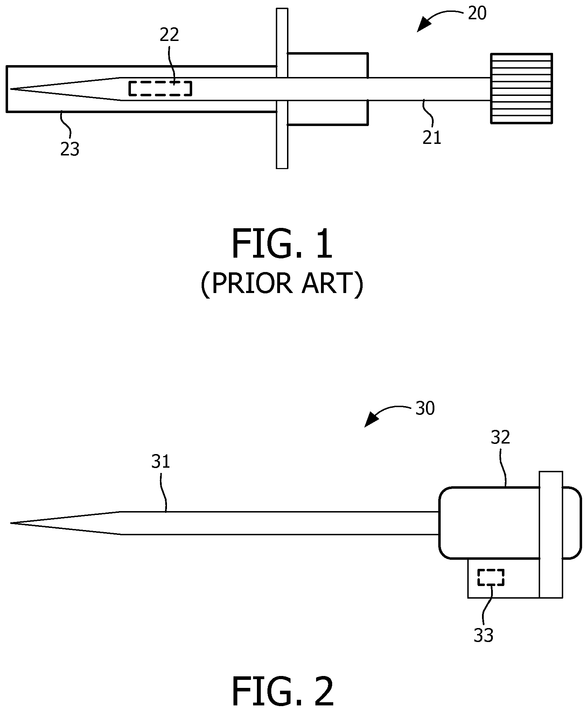

The first approach involves a position tracker built to a co-axial introducer system as shown in FIG. 1. Specifically, a tracked introducer system 20 employs a stylet 21 having a position sensor 22 and a cannula 23. Cannula 23 serves as a channel that is used to host stylet 21, which serves as a tracked needle that is used as the introducer of cannula 23 within an anatomical region. Cannula 23 is guided to a desired target anatomical location by mating with stylet 21, and sensor 22 provides tip position information of cannula 22. Once cannula 23 is placed in the desired anatomical location, stylet 21 is withdrawn and cannula 23 is positioned relative to the target anatomical location. A required instrument is introduced to the target anatomical location by inserting the instrument through cannula 23. The disadvantage is that the tip of cannula 23 can no longer be tracked once stylet 21 is pulled out of cannula 23. Cannula 23 therefore needs to be kept in place and assumed to remain at the target anatomical location. In addition, the instrument is not always compatible with cannula 23, and the cost is high because the introducer system is disposable. Lastly, with the introduction of cannula 23, the diameter of the insertion into the anatomical region inevitably increases. Such a diameter increase is not desirable for cosmetic reasons and is not recommended in many clinical situations.

As shown in FIG. 2, the second approach involves a hub tracker 32 having a position sensor 33 with hub tracker 32 being designed to attach to a proximal hub of a shaft 31 of an instrument 30. Hub tracker 32 may be designed to universally fit typical instruments and once fitted on instrument 30, a calibration is required to determine the offset distance from a distal tip of shaft 31 to hub tracker 32 whereby position information of the distal tip of shaft 31 may be tracked.

The advantage of hub tracker 32 is that it may be compatible with many different instruments and not be limited by gauge size and length. In addition, since hub tracker 32 is outside the patient body, hub tracker 32 does not interfere with the operation of the instrument (e.g. thermal ablation of the tumor) and does not increase the insertion size of the introducer system 20 of FIG. 1. The disadvantage is that position sensor 33 is located away from the distal tip of shaft 31 resulting in the accuracy being sub-optimal due in view of any bending of shaft 31. In addition, a calibration step by the user is required after attaching hub tracker 32 to the proximal hub of shaft 31. Furthermore, although a hub tracker 32 may be designed to fit shaft 31 thereby eliminating the calibration step, such a design ties hub tracker 32 to shaft 31 whereby hub tracker may not be universally used with other instruments.

US 2007/0016009 A1 discloses an array for use with a surgical navigation system, wherein the array comprises a frame and first, second and third trackers attached to the frame, wherein the first trackers are moveable relative to the frame.

US 2002/0143317 A1 discloses an apparatus having an insertable portion for holding a position sensor, wherein the position sensor can transmit signals indicative of its position with respect to a field generator and the insertable portion of the catheter has fiducial markings that are detectable by an imaging modality when the insertable portion is inserted into an anatomical body.

US 2010/0210939 A1 discloses a surgical navigation system for tracking an instrument relative to a patient, wherein the system can track a portion of the patient, an instrument and/or both relative to image data, a coordinate system, an atlas, a morphed atlas, or combinations thereof.

DE 10 2010 046 948 A1 discloses a surgical target device for positioning drill holes in bone comprising a guide arm, a target hook stretching away from the guide arm and a drill guide positioned on the guide arm, wherein the target device further comprises at least one marker and/or a sensor to provide position data of the target device.The present invention provides a shaft tracker integrated onto the shaft of the instrument and serving as a distal tip marker whereby the aforementioned disadvantages of the conventional designs are mitigated.

The present invention is an intervention instrument employing shaft and a shaft tracker. The shaft extends between a distal tip and a proximal hub, and the shaft tracker partially or completely encircles the shaft and is movable to a primary tracking position along the shaft between the distal tip and the proximal hub. The primary tracking position is derived from a distance from an entry point of the distal tip into an anatomical region to a target location of the distal tip within the anatomical region. The shaft tracker includes a primary position sensor operable for tracking the shaft tracker relative to the anatomical region at or offset from the primary tracking position. The intervention instrument further comprises an auxiliary tracker that at least partially encircles the shaft and is fixed at, or movable to, an auxiliary tracking position along the shaft between the shaft tracker and the proximal hub. The auxiliary tracker includes an auxiliary position sensor operable for tracking the auxiliary tracker relative to the anatomical region.

The foregoing forms and other forms of the present invention as well as various features and advantages of the present invention will become further apparent from the following detailed description of various embodiments of the present invention read in conjunction with the accompanying drawings. The detailed description and drawings are merely illustrative of the present invention rather than limiting, the scope of the present invention being defined by the appended claims,
FIG. 1 illustrates an exemplary embodiment of co-axial introducer system as known in the art.
FIG. 2 illustrates an exemplary embodiment of a hub tracker as known in the art.
FIG. 3 illustrates an exemplary embodiment of a shaft tracking system in accordance with the present invention.
FIG. 4 illustrates a flowchart representative of an exemplary embodiment of a shaft tracking method.
FIG. 5 illustrates an exemplary embodiment of a shaft tracker.
FIGS. 6-8 illustrate an exemplary interventional implementation of the shaft tracker shown in FIG. 5 in accordance with the flowchart shown in FIG. 3.
FIG. 9 illustrates an embodiment of a shaft tracker in accordance with the present invention.
FIGS. 10-12 illustrate an interventional implementation of the shaft tracker shown in FIG. 9 in accordance with the flowchart shown in FIG. 3.

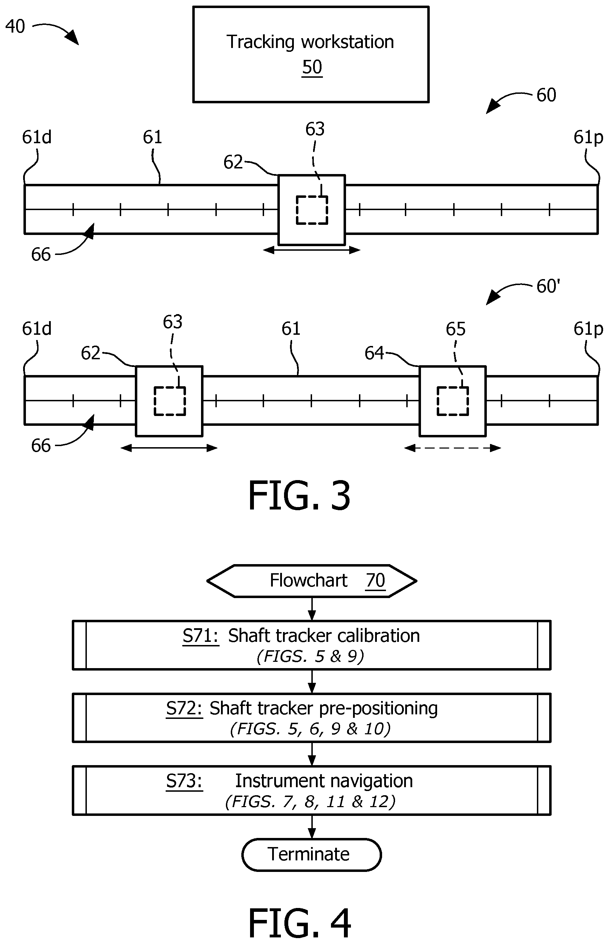

An intervention system 40 as shown in FIG. 3 employs a tracking workstation 50 and an intervention instrument 60.

Tracking workstation 50 is any workstation structurally configured for registering an intra-operative real-time imaging modality (e.g., x-ray, endoscope or ultrasound) with a pre-operative imaging modality (e.g., computed tomography or magnetic resonance imaging) via the aid of a tracking workstation (e.g., an EM tracking workstation or an optical tracking workstation). As would be appreciated by those having skill in the art, the purpose of the image registration is to utilize the pre-operative image and/or the intra-operative image of an anatomical region as a visual guide for the intra-operative navigation of interventional instrument 60 from an entry point into an anatomical region to a target location within the anatomical region. To this end, tracker workstation includes position sensor(s) incorporated in instrument 60 as subsequently described herein. An example of tracking workstation 50 includes, but is not limited to, a PERCUNAV^{™} image fusion and navigation device as commercially sold by Philips.

Intervention instrument 60 is any instrument structurally configured with shaft 61 having a distal tip 61d and a proximal hub 61p, and a shaft tracker 62 partially or completely encircling shaft 61 between distal tip 61d and proximal hub 61p. Shaft tracker 62 is moveable along shaft 61 between distal tip 61d and proximal hub 61p to a primary tracking position identified by an optional distance scale 66 of specified increments (e.g., 1 mm increments). Shaft tracker 62 incorporates a primary position sensor 63 (e.g., an electromagnetic coil from the EM tracking workstation or an optical marker from the optical tracking workstation) that provides tracking of the shaft tracker 62 relative to the anatomical region at or offset from the primary tracking position.

In practice, shaft 61 may have any size and shape, and be constructed from any material suitable for a particular minimally invasive intervention or surgery (e.g., a needle, a cannula, a guide wire, etc.)

A modified version 60' of intervention instrument 60 is structurally configured with an auxiliary tracker 64 partially or completely encircling shaft 61 between distal tip 61d and proximal hub 61p, in accordance with the invention. Auxiliary tracker 64 is moveable along shaft 61 between shaft tracker 62 and proximal hub 61p to an auxiliary tracking position identified by optional distance scale 65. Alternatively, auxiliary tracker 64 may be fixed at the auxiliary tracking position. Auxiliary tracker 64 incorporates an auxiliary position sensor 65 (e.g., an electromagnetic coil from the EM tracking workstation or an optical marker from the optical tracking workstation) that provides tracking of the auxiliary tracker 64 relative to the anatomical region at or offset from the auxiliary tracking position.

In operation, system 40 executes a shaft tracking method as represented by a flowchart 70 shown in FIG. 4.

Specifically, a stage S71 of flowchart 70 encompasses an optional calibration of shaft tracker 62 relative to distal tip 61d of shaft 60 when a high tracking accuracy is required. In one embodiment of stage S71, an estimated offset of shaft sensor 62 to distal tip 61d is calibrated as needed to an actual offset of shaft sensor 62 to the distal tip 61d. For example, a pivoting tool that allows carrying out this calibration under sterile conditions has a simple tracked surface with a pivot point cut into it. The distal tip 61d of shaft 61 is placed into the pivot point to measure the sensed distance between shaft tracker 62 and distal tip 61d of shaft 60. In practice, the pivoting tool is sterilizable and reusable. Since shaft tracker 62 may be made to universally fit onto intervention instrument of different gauge sizes, a centering mechanism may be utilized to account for a possible lateral offset by keeping shaft 61 at any gauges always at the center of shaft tracker 62. Alternatively, a programming step may be used whereby the user enters the gauge of instrument 60 and then software may then account for the resulting off-axis shift.

Auxiliary tracker 64, as employed by intervention instrument 60 according to the invention, may be similarly calibrated if needed.

A stage S72 of flowchart 70 encompasses a pre-positioning of shaft tracker 62 along shaft 61 relative to the primary tracking position that is derived a distance from an entry point of the distal tip 61d into an anatomical region to a target location of the distal tip 61d within the anatomical region. For example, the distance from an entry point of the distal tip 61d into an anatomical region to a target location of the distal tip 61d within the anatomical region may be X mm and the primary tracking position is determined to be ≥ X mm.

In a pre-operative locking embodiment of stage S72, an anatomical region of the patient is known via pre-operative images whereby the distance of a target location from an entry point is known. Prior to insertion of interventional instrument 60 into the entry point, shaft tracker 62 is moved and locked to the primary tracking position via scale 66 or via a manual measurement from the distal tip 61d. For this embodiment, a stage S73 of flowchart 70 encompasses a navigation of intervention instrument 60 into the entry point until such time shaft tracker 62 abuts the entry point or is substantially adjacent the entry point. Based on the locked primary tracking position of shaft tracker 62, the distal tip 61d of shaft 61 will have reached the target location upon shaft tracker 62 abutting the entry point.

In an intra-operative movement embodiment of stage S72 with intervention instrument employing auxiliary tracker 64, the anatomical region of the patient is also known via pre-operative images whereby the distance of the target location from the entry point is known. Prior to insertion of interventional instrument 60 into the entry point, shaft tracker 62 is moved to the distal tip 61d of shaft 61 and kept unlocked and auxiliary tracker 64 is moved to the auxiliary tracking position and is locked. Alternatively, auxiliary tracker 64 may be fixed at the auxiliary tracking position. For this embodiment, stage S73 of flowchart 70 encompasses a navigation of intervention instrument 60 into the entry point whereby shaft tracker 62 abuts the entry point and is moved along shaft 61 in a direction toward the primary tracking position. Intervention instrument 60 is navigated until such time the distance between shaft tracker 62 and auxiliary tracker 64 indicates shaft tracker 62 has been moved to the primary tracking position. Based on shaft tracker 62 reaching the primary tracking position, the distal tip 61d of shaft 61 will have reached the target location.

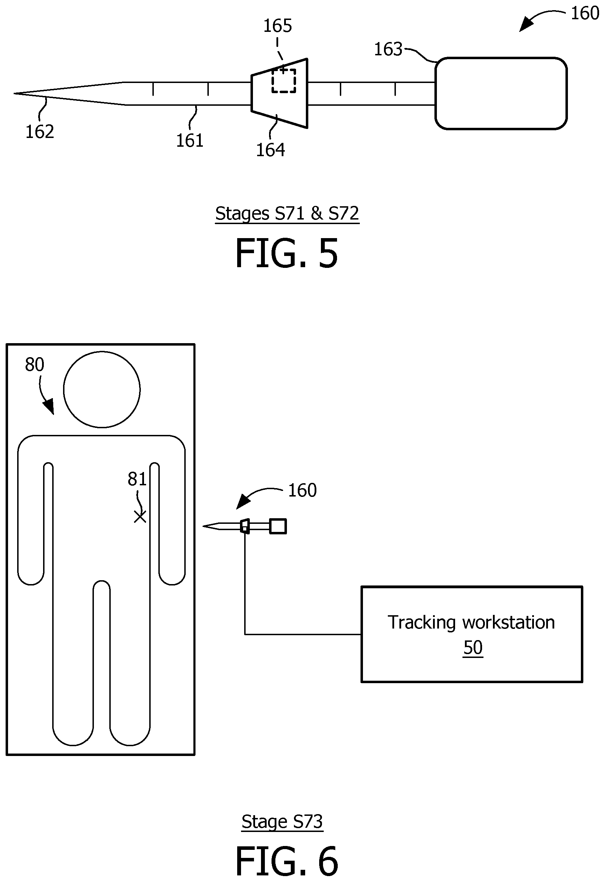

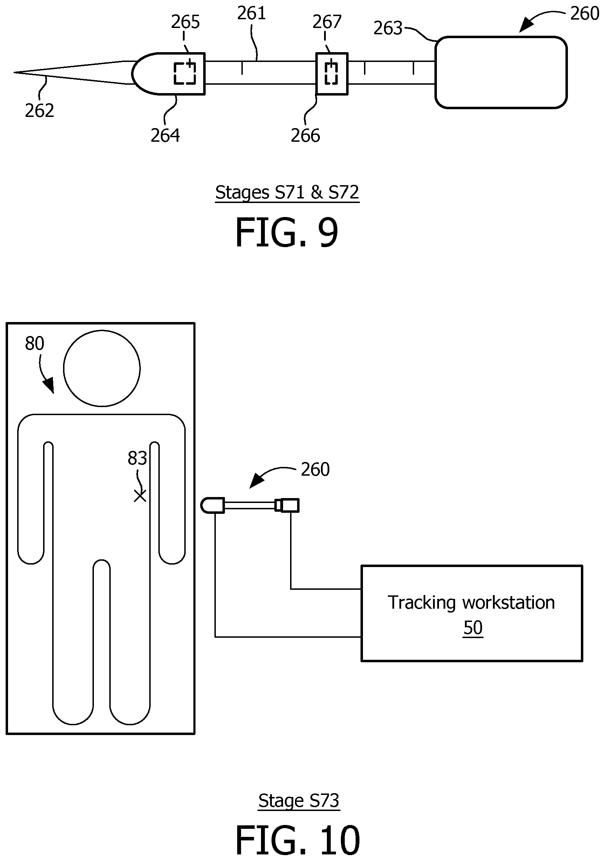

Exemplary embodiments 160 and 260 of intervention instrument 60 as respectively shown in FIGS. 5 and 9 will now be described herein.

As shown in FIG. 5, intervention instrument 160 is a needle structurally configured with shaft 161 having a distal tip 162 and a proximal hub 163, and a shaft tracker 164 completely encircling shaft 161 between distal tip 162 and proximal hub 163. Shaft tracker 164 is moveable along shaft 161 between distal tip 162 and proximal hub 163 to a shaft position identified by a distance scale of specified increments (e.g., 1 mm increments). Shaft tracker 164 incorporates a primary position sensor 165 (e.g., an electromagnetic coil from the EM tracking workstation or an optical marker from the optical tracking workstation) that provides tracking of the shaft position of shaft tracker 164 relative to the anatomical region at or offset from the primary tracking position.

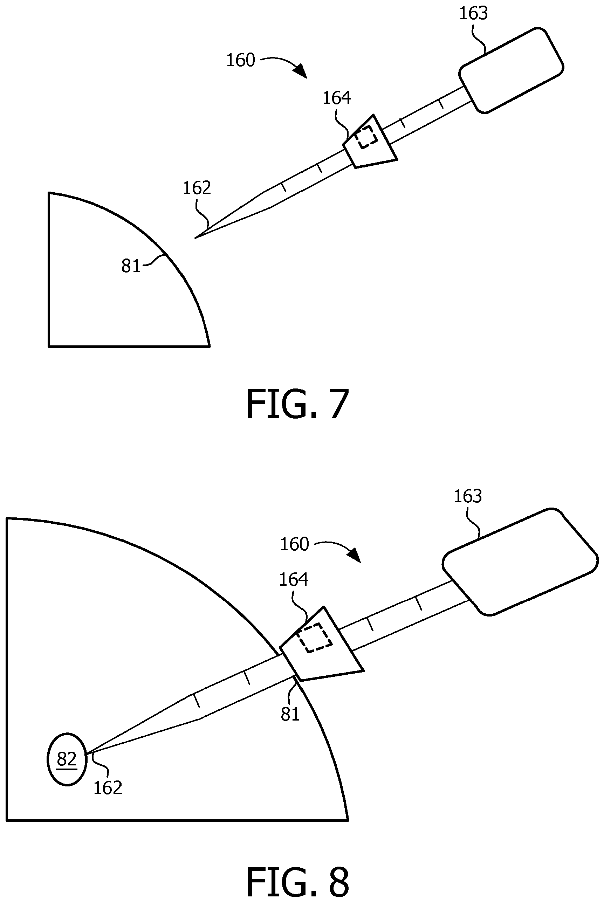

In preparation, an anatomical region of the patient is known via pre-operative images whereby a distance of a target location from an entry point is known. Prior to insertion of interventional instrument 160 into the entry point, shaft tracker 164 is moved and locked to a primary tracking position via scale 66 or via a manual measurement from the distal tip 61d as shown in FIG. 6. In operation, intervention instrument 160 is navigated into an entry point 81 of a patient 80 as shown in FIG. 7 until such time shaft tracker 164 abuts the entry point as shown in FIG. 8 or is adjacent entry point 81. Based on the locked primary tracking position of shaft tracker 164, the distal tip 162 of shaft 161 will have reached a target location 82 upon shaft tracker 164 abutting the entry point 81.

As shown in the embodiment according to the invention in FIG. 9, intervention instrument 260 is a needle structurally configured with shaft 261 having a distal tip 262 and a proximal hub 263, a shaft tracker 264 completely encircling shaft 261 between distal tip 262 and auxiliary tracker 266 completely encircling shaft 261 between shaft tracker 264 and proximal hub 263. Shaft tracker 264 is moveable along shaft 261 between distal tip 262 and auxiliary tracker 266 to a shaft position identified by a distance scale of specified increments (e.g., 1 mm increments). Shaft tracker 264 incorporates a primary position sensor 265 (e.g., an electromagnetic coil from the EM tracking workstation or an optical marker from the optical tracking workstation) that provides tracking of shaft tracker 264 relative to the anatomical region at or offset from the primary tracking position.

Auxiliary tracker 266 is moveable along shaft 261 between shaft tracker 264 and proximal hub 263 to an auxiliary tracking position identified by the distance scale. Alternatively, as shown in FIG. 10, auxiliary tracker 266 is fixed along shaft 261 adjacent proximal hub 263. As shown in FIG. 9, auxiliary tracker 266 incorporates an auxiliary position sensor 267 (e.g., an electromagnetic coil from the EM tracking workstation or an optical marker from the optical tracking workstation) that provides tracking of auxiliary tracker 266 relative to the anatomical region at or offset from the auxiliary tracking position.

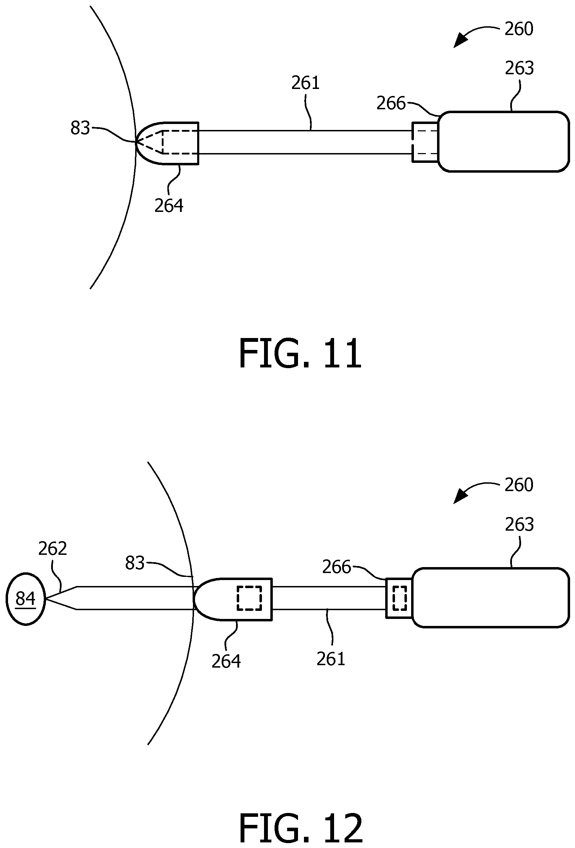

In preparation, the anatomical region of the patient is known via pre-operative images whereby a distance of the target location from the entry point is known. Prior to insertion of interventional instrument 260 into an entry point 83 as shown in FIG. 10, shaft tracker 264 (FIG. 9) is moved to the distal tip 262 of shaft 261 and kept unlocked and auxiliary tracker 266 is moved to the auxiliary tracking position and locked. Alternatively, auxiliary tracker 266 may be fixed at the auxiliary tracking position. In operation, intervention instrument 260 is navigated into entry point 83 (FIG.'s 11, 12) whereby shaft tracker 264 abuts entry point 83 and is moved along shaft 261 in a direction toward the primary tracking position derived from the distance of a target location 84 (FIG. 12) from entry point 83. Intervention instrument 260 is navigated until such time the distance between shaft tracker 264 and auxiliary tracker 266 indicates shaft tracker 264 has been moved to the primary tracking position. Based on shaft tracker 264 reaching the primary tracking position, distal tip 262 of shaft 261 will have reached the target location 84.

From the description of FIGS. 1-12 herein, those having ordinary skill in the art will appreciate the numerous benefits of the present invention.

One exemplary benefit is the omission of a calibration stage unless a very high precision and accuracy is required. Specifically, since the insertion distal tip is predetermined and the shaft tracker is pre-operatively or intra-operatively moved to the primary tracking position, this inherently provides the tip offset distance that is required to track the tip position without any need for calibration of the shaft tracker to the distal tip.

A second exemplary benefit is the shaft tracker will not interfere with the operation of the intervention instrument since the shaft tracker remains outside the patient's body and also does not increase the size insertion hole size for the intervention instrument. As a result, the shaft track will not possess any issue of cancer seeding along the instrument shaft.

A third exemplary benefit is an increased accuracy and minimized inaccuracy due to bending of the instrument in view of procedures involving the shaft tracker being position closer to the distal tip as compared to the proximal hub.

A fourth exemplary benefit is the universal fit of a shaft tracker onto intervention instruments of different gauge sizes and the independence on the shaft tracker to a design of the instrument handle.

Although the present invention has been described with reference to exemplary aspects, features and implementations, the disclosed systems are not limited to such exemplary aspects, features and/or implementations. Rather, as will be readily apparent to persons skilled in the art from the description provided herein, the disclosed systems are susceptible to modifications, alterations and enhancements without departing from the scope of the present invention as defined by the appended claims.

## Claims

1. An intervention instrument (60), comprising:
a shaft (61) extending between a distal tip and a proximal hub; and
a shaft tracker (62) at least partially encircling the shaft (61) and movable to a primary tracking position along the shaft (61) between the distal tip and the proximal hub,
wherein the primary tracking position is derived from a distance from an entry point of the distal tip into an anatomical region to a target location of the distal tip within the anatomical region, and
wherein the shaft tracker (62) includes a primary position sensor (63) operable for tracking the shaft tracker (62) relative to the anatomical region; **characterised in that** the intervention instrument (60) further comprises an auxiliary tracker (64) at least partially encircling the shaft (61) and fixed at, or movable to, an auxiliary tracking position along the shaft (61) between the shaft tracker (62) and the proximal hub,
wherein the auxiliary tracker (64) includes an auxiliary position sensor (65) operable for tracking the auxiliary tracker (64) relative to the anatomical region.

2. The intervention instrument (60) of claim 1, wherein the primary position sensor (63) is selected from a group including an electromagnetic coil and an optical marker.

3. The intervention instrument (60) of clam 1, wherein the shaft (61) includes a distal tip scale (66) representative of incremental distances between the distal tip and the proximal hub.

4. The intervention instrument (60) of claim 1, wherein the auxiliary position sensor (65) is selected from a group including an electromagnetic coil and an optical marker.

5. An intervention system (40), comprising:
an intervention instrument (60) according to any one of claims 1 to 4 and
a tracking workstation (50) operable for tracking the primary position sensor (63) relative to the anatomical region.

6. The intervention system (40) of claim 5, wherein the tracking workstation (50) is further operable for tracking the auxiliary position sensor (65) relative to the anatomical region.

7. The intervention system (40) of claim 5 or 6, wherein, responsive to a tracking the shaft tracker (62) relative to the anatomical region, the tracking workstation (50) is operable to monitor a navigation of the distal tip of shaft (61) from the entry point into the anatomical region to the target location within the anatomical region as illustrated within an image of the anatomical region.

## Patentansprüche

1. Interventionsinstrument (60), umfassend:
einen Schaft (61), der sich zwischen einer distalen Spitze und einer proximalen Nabe erstreckt; und
einen Schaftverfolger (62), der den Schaft (61) zumindest teilweise umgibt und in eine primäre Verfolgungsposition entlang des Schafts (61) zwischen der distalen Spitze und der proximalen Nabe bewegt werden kann,
wobei die primäre Verfolgungsposition aus einem Abstand von einem Eintrittspunkt der distalen Spitze in einen anatomischen Bereich zu einer Zielposition der distalen Spitze innerhalb des anatomischen Bereichs abgeleitet wird, und wobei der Schaftverfolger (62) einen primären Positionssensor (63) umfasst, der zum Verfolgen des Schaftverfolgers (62) in Bezug auf den anatomischen Bereich betrieben werden kann; **dadurch gekennzeichnet,**
**dass** das Interventionsinstrument (60) ferner einen Hilfsverfolger (64) umfasst, der den Schaft (61) zumindest teilweise umgibt und an einer Hilfsverfolgungsposition entlang des Schafts (61) zwischen dem Schaftverfolger (62) und der proximalen Nabe befestigt ist oder zu dieser bewegt werden kann, wobei der Hilfsverfolger (64) einen Hilfspositionssensor (65) beinhaltet, der zum Verfolgen des Hilfsverfolgers (64) in Bezug auf den anatomischen Bereich betrieben werden kann.

2. Interventionsinstrument (60) nach Anspruch 1, wobei der primäre Positionssensor (63) ausgewählt ist aus einer Gruppe, die eine elektromagnetische Spule und einen optischen Marker beinhaltet.

3. Interventionsinstrument (60) nach Anspruch 1, wobei der Schaft (61) eine Skala (66) für die distale Spitze beinhaltet, die repräsentativ für inkrementelle Abstände zwischen der distalen Spitze und der proximalen Nabe ist.

4. Interventionsinstrument (60) nach Anspruch 1, wobei der Hilfspositionssensor (65) ausgewählt ist aus einer Gruppe, die eine elektromagnetische Spule und einen optischen Marker beinhaltet.

5. Interventionssystem (40), umfassend:
ein Interventionsinstrument (60) nach einem der Ansprüche 1 bis 4 und eine Verfolgungsarbeitsstation (50), die zum Verfolgen des primären Positionssensors (63) in Bezug auf den anatomischen Bereich betrieben werden kann.

6. Interventionssystem (40) nach Anspruch 5, wobei die Verfolgungsarbeitsstation (50) ferner zum Verfolgen des Hilfspositionssensors (65) in Bezug auf den anatomischen Bereich betrieben werden kann.

7. Interventionssystem (40) nach Anspruch 5 oder 6, wobei die Verfolgungsarbeitsstation (50) als Reaktion auf ein Verfolgen des Schaftverfolgers (62) in Bezug auf den anatomischen Bereich betrieben werden kann, um eine Navigation der distalen Spitze des Schafts (61) von dem Eintrittspunkt in den anatomischen Bereich zu dem Zielort innerhalb des anatomischen Bereichs zu überwachen, wie in einem Bild des anatomischen Bereichs veranschaulicht ist.

## Revendications

1. Instrument d'intervention (60), comprenant:
un arbre (61) s'étendant entre une pointe distale et un moyeu proximal; et
un suiveur d'arbre (62) encerclant au moins partiellement l'arbre (61) et mobile vers une position de suivi primaire le long de l'arbre (61) entre la pointe distale et le moyeu proximal,
dans lequel la position de suivi primaire est dérivée d'une distance entre un point d'entrée de la pointe distale dans une région anatomique et un emplacement cible de la pointe distale à l'intérieur de la région anatomique, et dans lequel le suiveur d'arbre (62) comprend un capteur de position principal (63) utilisable pour suivre le suiveur d'arbre (62) par rapport à la région anatomique; **caractérisé en ce que** l'instrument d'intervention (60) comprend en outre un suiveur auxiliaire (64) encerclant au moins partiellement l'arbre (61) et fixé à, ou mobile vers,
une position de suivi auxiliaire le long de l'arbre (61) entre le suiveur d'arbre (62) et le moyeu proximal, dans lequel le suiveur auxiliaire (64) comprend un capteur de position auxiliaire (65) utilisable pour suivre le suiveur auxiliaire (64) par rapport à la région anatomique.

2. Instrument d'intervention (60) selon la revendication 1, dans lequel le capteur de position principal (63) est sélectionné dans un groupe comprenant une bobine électromagnétique et un marqueur optique.

3. Instrument d'intervention (60) selon la revendication 1, dans lequel l'arbre (61) comprend une échelle de pointe distale (66) représentative des distances incrémentielles entre la pointe distale et le moyeu proximal.

4. Instrument d'intervention (60) selon la revendication 1, dans lequel le capteur de position auxiliaire (65) est sélectionné dans un groupe comprenant une bobine électromagnétique et un marqueur optique.

5. Système d'intervention (40), comprenant:
un instrument d'intervention (60) selon l'une quelconque des revendications 1 à 4 et
un poste de travail de suivi (50) utilisable pour suivre le capteur de position principal (63) par rapport à la région anatomique.

6. Système d'intervention (40) selon la revendication 5, dans lequel la station de travail de suivi (50) est en outre utilisable pour suivre le capteur de position auxiliaire (65) par rapport à la région anatomique.

7. Système d'intervention (40) selon la revendication 5 ou 6, dans lequel, en réponse à un suivi du suiveur d'arbre (62) par rapport à la région anatomique, le poste de travail de suivi (50) peut fonctionner pour surveiller une navigation de la pointe distale de l'arbre (61) du point d'entrée dans la région anatomique à l'emplacement cible dans la région anatomique comme illustré dans une image de la région anatomique.
